(19) Europäisches Patentamt / European Patent Office / Office européen des brevets

(11) EP 1 363 932 B1

(12) EUROPÄISCHE PATENTSCHRIFT

(45) Veröffentlichungstag und Bekanntmachung des Hinweises auf die Patenterteilung:
**13.06.2007 Patentblatt 2007/24**

(21) Anmeldenummer: **02729931.2**

(22) Anmeldetag: **21.02.2002**

(51) Int Cl.:
*C07J 41/00* (2006.01) *A61K 9/127* (2006.01)

(86) Internationale Anmeldenummer:
**PCT/EP2002/001878**

(87) Internationale Veröffentlichungsnummer:
**WO 2002/066489 (29.08.2002 Gazette 2002/35)**

# (54) AMPHOTERE STEROLE UND DEREN VERWENDUNG

AMPHOTERIC STEROLS AND THE USE THEREOF

STEROLS AMPHOTERES ET LEUR UTILISATION

(84) Benannte Vertragsstaaten:
**AT BE CH CY DE DK ES FI FR GB GR IE IT LI LU MC NL PT SE TR**

(30) Priorität: **21.02.2001 DE 10109898**

(43) Veröffentlichungstag der Anmeldung:
**26.11.2003 Patentblatt 2003/48**

(60) Teilanmeldung:
**07106269.9**

(73) Patentinhaber: **novosom AG**
**06120 Halle (DE)**

(72) Erfinder:
- **PANZNER, Steffen**
  **06114 Halle (DE)**
- **ENDERT, Gerold**
  **06114 Halle (DE)**
- **FANKHÄNEL, Stefan**
  **04451 Borsdorf (DE)**
- **EL-MOKDAD, Nasr**
  **06110 Halle (DE)**

(74) Vertreter: **Ziebig, Marlene et al**
**Wallstrasse 58/59**
**10179 Berlin (DE)**

(56) Entgegenhaltungen:

| | |
|---|---|
| **WO-A-00/09073** | **WO-A-00/30688** |
| **WO-A-00/59474** | **WO-A-96/11023** |
| **WO-A-98/43994** | **US-A- 5 993 823** |

- **LI, ZI CHEN ET AL: "Synthesis of cholesterol derivatives with amino acid as hydrophilic group and the vesicles prepared therefrom" CHINESE CHEMICAL LETTERS (1999), 10(12), 1007-1010 , XP008005314**
- **NASTRUZZI C ET AL: "Liposomes as carriers for DNA-PNA hybrids" JOURNAL OF CONTROLLED RELEASE, ELSEVIER SCIENCE PUBLISHERS B.V. AMSTERDAM, NL, Bd. 68, Nr. 2, 10. August 2000 (2000-08-10), Seiten 237-249, XP004228954 ISSN: 0168-3659**
- **DATABASE CA [Online] CHEMICAL ABSTRACTS SERVICE, COLUMBUS, OHIO, US; ISHIKURA, TOYOAKI ET AL: "Preparation of peptide-lipid derivatives and liposomes containing the derivatives" retrieved from STN Database accession no. 122:161385 XP002204265 & JP 06 219967 A (DDS KENKYUSHO KK, JAPAN) 9. August 1994 (1994-08-09)**
- **DATABASE CA [Online] CHEMICAL ABSTRACTS SERVICE, COLUMBUS, OHIO, US; LANDAU, EHUD M. ET AL: "Transfer of structural information from Langmuir monolayers to three-dimensional growing crystals" retrieved from STN Database accession no. 104:99668 XP002204266 & NATURE (LONDON) (1985), 318 (6044), 353-6 ,**
- **DATABASE CA [Online] CHEMICAL ABSTRACTS SERVICE, COLUMBUS, OHIO, US; PLEURDEAU, A. ET AL: "Synthesis of polymers with pharmacological properties. Introduction of peptide sequences into the macromolecular chain" retrieved from STN Database accession no. 96:69405 XP002204267 & EUR. POLYM. J. (1981), 17(9), 999-1003 ,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

- BUDKER V ET AL: "PH-SENSITIVE, CATIONIC LIPOSOMES: A NEW SYNTHETIC VIRUS-LIKE VECTOR" BIO/TECHNOLOGY, NATURE PUBLISHING CO. NEW YORK, US, Bd. 14, Juni 1996 (1996-06), Seiten 760-764, XP002937150 ISSN: 0733-222X
- DATABASE CA [Online] CHEMICAL ABSTRACTS SERVICE, COLUMBUS, OHIO, US; HAFEZ, ISMAIL M. ET AL: "Tunable pH - sensitive liposomes composed of mixtures of cationic and anionic lipids" retrieved from STN Database accession no. 133:355087 XP002204268 & BIOPHYSICAL JOURNAL (2000), 79(3), 1438-1446 ,
- LI, XIAO-JUN ET AL: "Theory of tunable pH sensitive vesicles of anionic and cationic lipids or anionic and neutral lipids" LOS ALAMOS NATIONAL LABORATORY, PREPRINT ARCHIVE, CONDENSED MATTER (2000) 1-16, ARXIV: COND-MAT/0009267, 22 SEP 2000 , [Online] 22. September 2000 (2000-09-22), XP002204264 Gefunden im Internet: <URL:http: //xxx.lanl.gov/pdf/cond-mat/0009267> [gefunden am 2002-06-28]

## Beschreibung

Die Erfindung betrifft amphotere Verbindungen auf Basis eines Sterolgerüstes nach der allgemeinen Formel (1)

$$\text{Amphoter} - Y - \text{Spacer} - X - \text{Sterol} \quad (1)$$

sowie Liposomen, die diese Verbindungen enthalten.

Unter dem Begriff der Lipide werden drei Klassen von Naturstoffen zusammengefasst, die sich aus biologischen Membranen isolieren lassen: Phospholipide, Sphingolipide und Cholesterol mit seinen Derivaten.

Von technischem Interesse sind diese Substanzen bei der Herstellung von Liposomen. Diese Liposomen lassen sich unter anderem als Container für Wirkstoffe bei pharmazeutischen Zubereitungen einsetzen. Wünschenswert ist dabei eine effiziente und stabile Verpackung des Cargos und eine kontrollierbare Freisetzung des Inhalts. Beide Ansprüche sind nicht ohne weiteres zu vereinen: Je stabiler und dichter die Verpackung ist, desto schwerer gibt sie den eingeschlossenen Wirkstoff wieder frei. Aus diesem Grund wurden Liposomen entwickelt, die ihre Eigenschaften als Reaktion auf einen äußeren Reiz verändern. Bekannt sind thermosensible und pH-sensitive Liposomen. Die pH-sensitiven Liposomen sind von besonderem Interesse, da dieser Parameter sich auch unter physiologischen Umständen, etwa bei der endozytotischen Aufnahme eines Liposoms in Zellen oder bei der Passage des Magen-Darm-Trakts, ändern kann. Nach dem Stand der Technik umfassen pH-sensitive Liposomen insbesondere Cholesterolhemisuccinat (CHEMS).

Cholesterolhemisuccinat wird in Mischung mit Phosphatidylethanolamin zur Herstellung pH-sensitiver Liposomen verwendet (Tachibana et al.(1998); BBRC 251: 538-544, US4891208). Solche Liposomen können von Zellen endozytiert werden und vermögen auf diesem Weg Cargomoleküle in das Innere von Zellen zu transportieren, ohne die Integrität der zellulären Membran zu verletzen.

Ein wesentlicher Nachteil des CHEMS ist dessen anionischer Charakter. Die damit hergestellten Liposomen besitzen eine negative Gesamtladung und werden nur mit geringer Effizienz von Zellen aufgenommen. Trotz des oben beschriebenen Transfermechanismus eignen sie sich daher kaum für den Eintransport von Makromolekülen in Zellen.

Der Stand der Technik beschreibt in der WO 00/59474 Verbindungen mit einem Membrananker und einer kationischen und anionischen Kopfgruppe, wobei die anionische Gruppe über eine Disulfidbrücke mit der Grundstruktur verbunden ist. Diese Disulfidbrücke kann unter physiologischen Bedingungen, etwa bei Kontakt mit dem Cytosol, reduziert werden, die anionische Kopfgruppe wird dann freigesetzt und das Gesamtmolekül erhält eine positive Ladung und ermöglicht die Fusion mit der Zellmembran. Nachteilig ist das Toxizitätsprofil und die Lagerstabilität der in WO 00/59474 offenbarten Verbindungen, da durch die Abspaltung der Disulfidbrücken freie kationische Lipide entstehen. Für diese Verbindungen ist es bekannt, dass sie nachteilhafterweise eine zytotoxische Wirkung besitzen.

Für den Eintransport von Wirkstoffen in Zellen (Transfektion) werden fachgemäß kationische Liposomen verwendet, die über eine möglichst hohe und konstante Oberflächenladung verfügen. Die positive Gesamtladung solcher Partikel führt zu einer elektrostatischen Anheftung an Zellen und in der Folge zu einem effizienten Eintransport. Der Einsatz dieser Verbindungen und der damit hergestellten Liposomen bleibt aber auf Anwendungen in vitro oder ex vivo beschränkt, da solche positiv geladenen Liposomen mit Serumbestandteilen unkontrollierte Aggregate bilden.

Es bestand daher die Aufgabe, neue Verbindungen herzustellen,

i) mit deren Hilfe Wirkstoffe in Liposomen eingeschlossen und bei einer Veränderung des pH-Wertes wieder freigesetzt werden können,

ii) durch deren Anwesenheit die Herstellung von amphoteren Liposomen gelingt, die unter physiologischen Bedingungen mit Serum gemischt werden könne, ohne dass es zur Aggregation kommt und

iii) mit deren Hilfe Liposomen hergestellt werden können, die einen eingeschlossenen Wirkstoff in das Innere von Zellen transportieren können.

Eine weitere Aufgabe der Erfindung bestand darin, dass die Verbindungen sich einfach und preiswert herstellen lassen und in hohem Anteil in liposomale Membranen eingebaut werden können.

Die erfindungsgemäße Aufgabe wird gelöst durch Sterolderivate. Die Sterolderivate besitzen einen isoelektrischen Punkt zwischen 4,5 und 8,5 und nach der allgemeinen Formel mit den Merkmalen des Anspruchs 1.

$$\text{Amphoter} - Y - \text{Spacer} - X - \text{Sterol},$$

wobei Y und X wie folgt definiert sind.

Die erfindungsgemäße Aufgabe wird durch Konjugation von amphoteren Gruppen an die 3-Position eines Sterolgerüstes gelöst. Je nach verwendetem Amphoter werden Verbindungen erhalten, die bei einem spezifischen pH-Wert ihre Ladung ändern und sich überraschenderweise zu einem hohen Anteil in liposomale Membranen einbauen lassen. Als Ausgangsverbindung können einfache und billige Sterole oder deren Derivate verwendet werden.

Im Zusammenhang mit der Erfindung sollen folgende Abkürzungen verwendet werden:

| | |
|---|---|
| CHEMS | Cholesterolhemisuccinat |
| PC | Phospatidylcholin |
| PE | Phosphatidylethanolamin |
| PS | Phosphatidylserin |
| Hist-Chol | Histidinyl-cholesterolhemisuccinat |

Unter den membranbildenden oder membranständigen Gruppen einer biologischen Bilayermembran sind die Sterole von besonderem Interesse, da diese Verbindungen insbesondere billig verfügbar sind, eine einfache Chemie aufweisen und in einem hohen Anteil in Membranen eingebaut werden können, ohne deren Permeabilität zu erhöhen oder gar den Membrancharakter völlig zu zerstören. Wichtig für den Erhalt der letztgenannten Charakteristik ist jedoch, dass die Substitution mit einem polaren Molekül an der 3-Position des Sterols erfolgt.

Das Gesamtmolekül erhält seine pH-abhängige Ladungscharakteristik durch die gleichzeitige Anwesenheit von kationischen und anionischen Gruppen im Molekülteil "Amphoter". Ein Amphoter ist insbesondere dadurch gekennzeichnet, dass bei einem bestimmten pH-Wert die Summe seiner Ladungsbestandteile gerade Null ist. Dieser Punkt wird als isoelektrischer Punkt (pI) bezeichnet. Oberhalb des pI ist die Verbindung negativ geladen, unterhalb des pI ist sie als positives Kation aufzufassen; wobei der pI der erfindungsgemäßen Verbindungen bzw. Sterolderivate zwischen 4,5 und 8,5 liegt.

Die Gesamtladung des Moleküls bei einem bestimmten pH-Wert des Mediums kann wie folgt berechnet werden:

$$z = \Sigma ni * ((qi-1)+(10^{(pK-pH)}/(1+10^{(pK-pH)})))$$

qi absolute Ladung der ionischen Gruppe unterhalb ihres pK (Beispiel: Carboxyl =0, einfache Stickstoffbase = 1)
ni Anzahl dieser Gruppen im Molekül

Eine erfindungsgemäße Verbindung entsteht beispielsweise durch Kopplung der Aminogruppe des Histidins an Cholesterolhemisuccinat. Das Produkt ist bei einem neutralem pH-Wert von 7 negativ geladen, da die vorhandene Carboxylfunktion voll dissoziiert vorliegt und die Imidazolfunktion nur gering aufgeladen ist. Bei einem sauren pH-Wert von etwa 4 sind die Verhältnisse umgekehrt: die Carboxylfunktion ist nun weitgehend entladen, die Imidazolgruppe jedoch voll protoniert, die Gesamtladung des Moleküls ist daher positiv.

In einer bevorzugten Ausführungsvariante der Erfindung weist das Sterolderivat einen isoelektrischen Punkt zwischen 5 und 7 auf.

Erfindungsgemäß umfasst das Amphoter ein oder mehrere Kationen mit einem pKa-Wert zwischen 4 und 8 und gleichzeitig ein oder mehrere Anionen mit einem pKa-Wert zwischen 3 und 7. Eine vorteilhafte Auswahl von Art und Anzahl der funktionellen Gruppen kann anhand der oben genannten Formel vorgenommen werden.

Diese feststehenden Ladungen lassen sich dann durch die beschriebenen veränderlichen Ladungen überkompensieren. Das heißt, dass die veränderlichen Ladungsträger beispielsweise in einem Überschuss eingesetzt werden. Vorteilhaft bei der Verwendung voll dissoziierter Gruppen ist deren starke Polarität.

Amphotere können besonders bevorzugt als komplette Struktureinheiten vorliegen. Das ist beispielhaft der Fall bei der Imidazolcarbonsäure, oder der Imidazoldiessigsäure. Amphotere können insbesondere aus zwei Ladungsträgern zusammengesetzt sein, die beide im benannten pH-Bereich zwischen 4 und 9 ihre Ladung wechseln. Der gleichzeitig stattfindende Verlust an anionische Ladung und Gewinn an kationische Ladung führt zu einem Ladungswechsel des Gesamtmoleküls.

Erfindungsgemäß umfasst das Kation ein Imidazol, Piperazin, Morpholin, Purin und/oder Pyrimidin. Da die Stickstoffbasen als Ringsysteme vorliegen, existieren vorteilhafterweise Stellungsisomere, bei denen der verbindende Spacer an verschiedenen Positionen des organischen Kations substituiert ist. Durch die Stellungsisomerie lassen sich zweckmäßigerweise die pKa-Werte der organischen Kationen beeinflussen. Die dem zugrunde liegenden Regeln sind dem Fachmann bekannt. Alternativ können diese Einflüsse aus Tabellenwerken abgeschätzt werden (Handbook of Chemistry and Physics, 73. Band, S. 8 - 37 ff.).

Durch Ankopplung entstehen mit Vorteil amphiphile organische Kationen, die beispielsweise aus den folgenden Stoffklassen stammen:

2-, 3, 4- oder 5-substituierte Imidazole, 2-,3- oder 4- substituierte Morpholine, 1-, 2-, oder 3- substitutierte Piperazine, 3 - , 4 - , 5-, 6- oder 9-substituierte Purine, 2-, 4-, 5- oder 6-subsituierte Pyrimidine, Ganz besonders bevorzugt sind solche Molekülfragmente, wie sie in biologischen Systemen vorkommen, also beispielsweise 4-Imidazole (Histamine, Histidin selbst), 2-,6- oder 9- Purine (Adenine, Guanine, Adenosine oder Guanosine) oder 1-, 2- oder 4-Pyrimidine (Uracile, Thymine, Cytosine, Uridine, Thymidine, Cytidine). Die hier genannten Strukturfragmente können weitere Substituenten aufweisen. Das können beispielsweise Methyl-, Ethyl-, Propyl- oder Isopropylreste sein, besonders bevorzugt in hydroxylierte Form mit einer oder zwei Hydroxylgruppen. Das können aber auch Hydroxyl- oder Ketofunktionen des Ringsystems sein.

Erfindungsgemäß sind die anionischen Ladungsträger Carboxylgruppen. Selbstverständlich können alle Carbonsäuren als Ladungsträger eingesetzt werden. Dazu gehören insbesondere aliphatische, geradkettige oder verzweigte Carbonsäuren mit bis zu 8 C-Atomen und 0, 1 oder 2 ethylenisch ungesättigten Bindungen. Beispielhafte Verbindungsteile sind die Carboxylgruppe selbst, die in der aliphatischen Kette gebundene Essigsäure, Bromessigsäure, Chloressigsäure, Acetoessigsäure, Propionsäure, Acrylsäure, Buttersäure, Crotonsäure oder höhere Carbonsäure, die einfach veresterte oder amidierte oder in der aliphatischen Kette gebundene Dicarbonsäure wie Oxalsäure, Malonsäure, Bernsteinsäure, Maleinsäure, Fumarsäure, Äpfelsäure, Weinsäure, Glutarsäure, Adipinsäure, Caprylsäure, Pimelinsäure, Suberinsäure, Cyclohexandicarbonsäure oder auch Cyclopentandicarbonsäure; die einfach veresterte oder amidierte oder im aliphatischen Teil gebundene Oligocarbonsäure wie Citronensäure, Isocitronensäure oder Ethylendiamintetraessigsäure.

Weitere vorteilhafte Verbindungsteile sind die in der Seitenkette über ein Heteroatom gebundene Glykolsäure, Milchsäure, Hydroxybuttersäure, Äpfelsäure, Weinsäure, Asparginsäure oder Glutaminsäure, Alanin, Glycin, Serin, Threonin, Asparagin, Glutamin, Prolin, Tyrosin oder Cystein oder andere Aminosäure oder Hydroxysäure.

Carbonsäuren mit einem geeigneten Verhalten findet man auch als Substituenten aromatischen Systeme, etwa als Benzoesäure, Anissäure, o-, m- oder p-Hydroxybenzoesäure, als Dihydroxybenzoesäure, Gallussäure, Zimtsäure, Phenylessigsäure, Hippursäure, Pthalsäure, Terephtalsäure, 2,3 oder 4-Pyridincarbonsäure, Furancarboxylsäure. Andere anionische Gruppen sind dissoziierbare Hydroxyle oder Thiole, wie sie in der Ascorbinsäure, dem N-substituierten Alloxan, der N-substituierten Barbitursäure, im Veronal, dem Phenol oder als Thiolgruppe vorkommen.

Erfindungsgemäß sind die Sterole, Cholesterol, Sitosterol, Campesterol, Desmosterol, Fucosterol, 2.2-Ketosterol, 20-Hydroxysterol, Stigmasterol, 22-Hydroxycholesterol, 25-Hydroxycholesterol, Lanosterol, 7-Dehydrocholesterol, Dihydrocholesterol, 19-Hydroxycholesterol, 5α-Cholest-7-en-3β-ol, 7-Hydroxycholesterol, Epicholesterol, Ergosterol und/oder Dehydroergosterol sowie andere verwandte Verbindungen. Die mit Vorteil als Ausgangsstoff verwendeten Sterole können an ihrer 3-Position verschiedene Gruppen tragen, die zweckmäßigerweise eine einfache und beständige Ankopplung erlauben oder optional die Funktion eines räumlichen Spacers erfüllen. Besonders geeignet für eine direkte Kopplung sind die natürlicherweise vorhandene Hydroxylgruppe, aber auch das Chlor der Sterolchloride oder die Aminogruppe der Sterolamine oder die Thiolgruppe des Thiocholesterols.

Erfindungsgemäß umfasst die verbindende Gruppe X und Y die Struktur -(C=O)-O-; -(C=O)-NH-; -NH-(C=O)-O-; -(C=O)-S-; -O-; -NH-;-S- -CH=N--O-(O=C)-; -S-(O=C)-; -NH-(O=C)-; -O-(O=C)-NH- oder -N=CH- umfassen.

Erfindungsgemäß ist der Spacer ist ein Niederalkylrest mit linearer, verzweigter oder ringförmiger Struktur, der bis zu 8 C-Atome besitzt und 0, 1 oder 2 ethylenische ungesättigte Bindungen enthält. Der Spacer kann zur Erhöhung der Polarität des Moleküls Hydroxylgruppen besitzen.

Methoden zur Ausführung solcher Kopplungsreaktionen sind dem Fachmann bekannt und werden je nach verwendetem Ausgangsstoff und Kopplungskomponente variieren. Typische Reaktionen sind die Veresterung, die Amidierung, die Addition von Aminen an Doppelbindungen, die Veretherung oder auch die reduktive Aminierung.

Besonders bevorzugte Moleküle lassen sich durch Amidierung oder Veresterung von Cholesterolhemisuccinat oder durch die Bildung des Carbamoyls aus Cholesterolchloroformiat oder auch durch direkte Veresterung mit Cholesterol herstellen. Zu den besonders bevorzugten Amphoteren gehören beispielsweise die folgenden Verbindungen in Tab. 1, wobei R1 oder R2 den lipophilen Teil des amphoteren Sterols bedeuten und ( )n weitere Molekülteile im Sinne des oben definierten Spacers darstellen.

**Tabelle 1**

| | |
|---|---|
| 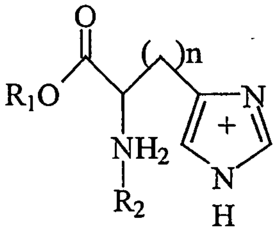 | Histidin-Derivate.<br>Bevorzugt erfolgt die Ankopplung des Sterols über die Aminogruppe als R2. R1 bildet in diesem Fall ein Anion und kann beispielsweise H oder eine Hydroxycarbonsäure oder eine oder mehrere Aminosäuren sein.<br>Erfolgt die Ankopplung über R1, dann ist R2 ein anionischer Rest, etwa eine Carbonsäure oder Dicarbonsäure. |

(fortgesetzt)

| | |
|---|---|
| 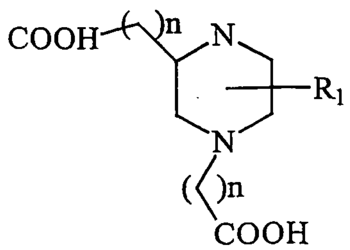 | Piperazin-Derivate.<br>Die Ankopplung des Sterols kann über eines der Ringatome erfolgen. Sind die Seitenketten als Hydroxycarbonsäuren oder Aminosäuren ausgeführt, so kann die Ankopplung vorteilhafterweise über diese Heteroatome erfolgen. |
| 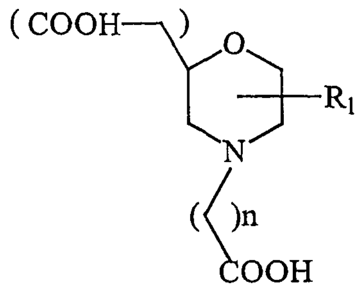 | Morpholin-Derivate<br>Die Ankopplung des Sterols kann über eines der Ringatome erfolgen. Sind die Seitenketten als Hydroxycarbonsäuren oder Aminosäuren ausgeführt, so kann die Ankopplung vorteilhafterweise über diese Heteroatome erfolgen. |
| 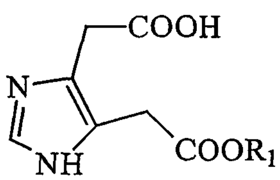 | Derivate der Imidazol-4,5-diessigsäure. Die Anbindung des Sterols erfolgt bevorzugt als Ester einer der beiden Essigsäurefunktionen. Das Sterols kann aber auch an die 3-Aminofunktion angebunden sein. |

Die Erfindung betrifft auch Liposomen, die die erfindungsgemäßen Sterolderivate umfassen. Die erfindungsgemäßen Verbindungen lassen sich in einem hohen Anteil in liposomale Membranen einbauen und führen erst dann zu einer positiven Ladung des Gesamtpartikels, wenn der pH-Wert des Mediums den isoelektrischen Punkt des Amphoters unterschreitet. Liposomen, umfassend die erfindungsgemäßen Komponenten können unter geeigneten Bedingungen mit Polymeren beschichtet werden. Dabei kann eine einfache oder mehrfache Abscheidung solcher Substanzen auf der Oberfläche erfolgen. Bei einer mehrfachen Abscheidung, die gegebenenfalls unter Anwesenheit von Vernetzer durchgeführt wird, entstehen liposomale Nanokapseln, wie sie in der WO 00/28972 oder in der WO01/64330 beschrieben sind. Vorteilhaft bei der Verwendung der hier beschriebenen Substanzen ist die Tatsache, dass die elektrostatische Interaktion mit dem Polyelektrolyten unterbrochen werden kann. Es ist bekannt, dass die Wechselwirkung eines Polyelektrolyten mit Ladungsträgern der liposomalen Membran zur Entmischung von Membranbestandteilen und zur Bildung von Lipidclustern führen kann. In vielen Fällen geht diese Entmischung mit einer Permeabilisierung des Liposoms einher. Die erfindungsgemäßen Substanzen ermöglichen eine Abschaltung dieser Wechselwirkung nach dem Beschichtungsprozess. Wird der pH-Wert zu diesem Zeitpunkt erhöht, so sind die Liposomen nur noch sterisch in der Nanokapseln eingeschlossen, eine Wechselwirkung der Membran mit den Polyelektrolyten besteht dann nicht mehr. Clusterbildung der Lipide und damit verbundene Permeabilisierung der Membran können so umgangen werden.

Es wurde überraschend gefunden, dass Liposomen, deren Membran die erfindungsgemäßen Substanzen enthalten, unterhalb des isoelektrischen Punktes der Substanz leicht mit anderen Membranen, insbesondere Zellmembranen fusionieren. Für gewöhnlich erfordert dieser Schritt die Anwesenheit eines größeren Anteils von PE in der Membran. Dieses hat durch seine Neigung zur Bildung von hexagonalen Phasen die Funktion eines Helferlipids. Nachteilig ist aber die geringere Stabilität solcher Membranen, hier wird oft eine schleichende Freisetzung von eingeschlossenen Wirkstoffen beobachtet.

Liposomen, die unter Verwendung der erfindungsgemäßen Substanzen hergestellt werden, fusionieren vorteilhafterweise effektiv in Abwesenheit von Helferlipiden. Es sind also unter Verwendung der erfindungsgemäßen Substanzen Liposomen herstellbar, die einen Wirkstoff stabil verkapseln können, aber unter den Bedingungen eines niedrigen pH-Werts mit Zellmembranen fusionieren und dort den Wirkstoff freisetzen.

Diese Kombination zweier Eigenschaften stellt eine wichtige Voraussetzung für die Einbringung von Cargomolekülen in Zellen dar. Bei einer Fusion von Liposomen mit Zellhüllen oder -bestandteilen vereinigen sich die wässrigen

Lumen beider Partner, ohne das es zu einer Öffnung der Membranstrukturen gegenüber dem Medium kommt. Dadurch wird der unkontrollierte Ein- oder Ausstrom anderer Substanzen vermieden.

In einer bevorzugten Ausführungsform der Erfindung umfasst der Anteil des Sterolderivats maximal 50 mol%. Besonders vorteilhaft sind Zusammensetzungen, die mindestens 2 mol%, höchstens aber 50 mol% der Sterolderivate umfassen. Weiter bevorzugt sind Zusammensetzungen, die mindestens 10 mol% und höchstens 40 mol% des Sterolderivats umfassen. Die Herstellung von Liposomen umfassend die erfinderischen Substanzen erfolgt nach den dem Fachmann bekannten Techniken.

In einer weiteren bevorzugten Ausführungsvariante der Erfindung umfassen die Liposomen Phosphatidylcholin, Phosphatidylethanolamin, Diacylglycerol, Tetraetherlipid und/oder PEG-Lipd. Da Cholesterole selbst keine Liposomen ausbilden können, ist der Zusatz eines weiteren Lipids vorteilhaft. Dieses Lipid kann jedes Phospholipid sein. Selbstverständlich ist es auch möglich, dass dieses Lipid auch ein Ceramid oder Sphingolipid ist. Zweckmäßig kann es sein, das Lipid im polaren Teil mit Polyethylenglykol zu modifizieren.

In einer bevorzugten Ausführungsvariante der Erfindung weisen die Liposomen eine mittlere Größe zwischen 50 und 1000 nm, bevorzugt zwischen 50 und 300 nm, besonders bevorzugt zwischen 60 und 130 nm auf.

Zweckmäßig ist es, in die Liposomen Wirkstoffe einzuschließen. Sie können z.B. in der Krebstherapie und zur Therapie schwerer Infektionen verwendet werden. Liposomendispersionen können dazu injiziert, infundiert oder implantiert werden. Sie verteilen sich danach im Blut oder in der Lymphe oder geben als Depot ihren Wirkstoff kontrolliert ab. Letzteres kann durch hochkonzentrierte Dispersionen erreicht werden, die als Gele vorliegen. Die Liposomen können auch für topische Anwendung auf der Haut eingesetzt werden. Sie können insbesondere dazu beitragen, dass verschiedene Wirkstoffe besser in die Haut eindringen können oder sogar durch die Haut in den Körper gelangen können. Weiterhin ist es möglich die Liposomen für den Gentransfer einzusetzen. Genetisches Material kann wegen seiner Größe und Ladung meist nicht ohne Hilfsmittel in Zellen gelangen. Dazu bedarf es geeigneter Träger wie z.B. Liposomen oder Lipid-Komplexen. Diese sollen zusammen mit der DNA effizient und möglichst gezielt in die betroffenen Zellen aufgenommen werden. Dazu werden zelleigene Transportmechanismen wie die Endozytose herangezogen. Die erfindungsgemäßen Liposomen sind selbstverständlich auch als Modellmembranen einsetzbar. Liposomen sind in ihrer prinzipiellen Struktur den Zellmembranen sehr ähnlich. Sie können daher als Membranmodelle eingesetzt werden, um die Permeationsgeschwindigkeit von Wirkstoffen durch Membranen oder die Membranbindung von Wirkstoffen zu quantifizieren. Besonders vorteilhaft ist es, wenn der Wirkstoff ein Protein, ein Peptid, eine DNA, eine RNA, ein antisense-Nukleotid und/oder ein Decoy-Nukleotid ist.

In einer weiteren Ausführungsvariante der Erfindung sind mindestens 80% des Wirkstoffes in den Liposomen eingeschlossen. Nicht eingebaute, außen anhaftende Cargomoleküle können wenn nötig durch einen einfache Erhöhung des pH-Wertes entfernt werden. Dieser Schritt ist immer dann notwendig, wenn nicht eingebaute Cargomoleküle zu einer Aggregation der Liposomen führen. Vorteilhaft bei der Verwendung der erfindungsgemäßen Komponenten ist die Tatsache, dass die eingeschlossenen Wirkstoffe nur für den Zeitraum des eigentlichen Einschlusses unter Bedingungen gebracht werden müssen, die eine Interaktion mit der Lipidschicht erlauben. Sobald die Lipidschicht in sich geschlossen bleibt, kann zu anderen Bedingungen gewechselt werden. Eine denkbare Inaktivierung von Wirkstoffen, insbesondere von Proteinen kann dadurch minimiert werden.

Die Erfindung betrifft auch Verfahren zur Wirkstoffbeladung von Liposomen, wobei ein Bindungs-pH-Wert zur Verkapselung benutzt wird und ein zweiter pH-Wert zur Abtrennung der nicht gebundenen Wirkstoffe verwendet wird.

Die Erfindung betrifft weiterhin ein Verfahren zur Wirkstoffbeladung von Liposomen, wobei die Liposomen bei einem bestimmten pH-Wert permeabilisiert und folgend verschlossen werden. Bevorzugt können insbesondere Permeabilitätsänderungen gezielt zur Beladung von Liposomen genutzt werden. Ein einzuschließender Wirkstoff kann dabei unter Bedingungen hoher Permeabilität ins Medium zugegeben werden. Anschließend werden Bedingungen geringer Permeabilität eingestellt. Damit verbleibt der Wirkstoff im Innern der Liposomen. Nicht eingeschlossener Wirkstoff kann dann gegebenenfalls abgetrennt werden. Eine solche Permeabilitätsänderung kann an Liposomen oder an liposomalen Nanokapseln herbeigeführt werden.

Die Erfindung betrifft auch die Verwendung der Liposomen in der Diagnostik und in Freisetzungssystemen. Die Liposomen können selbstverständlich auch in einem Detektionssystem verwendet werden. Insbesondere können die Liposomen mit Metallionen beladen werden, deren Fluoreszens durch die Chelatisierung verstärkt wird, also beispielsweise Terbium- oder Europiumionen. Liposomen für diese Anwendung können selbstverständlich spezifitätsbestimmende Komponenten enthalten, also z.B. Antikörper, Lektine, Selektine, Rezeptoren oder Hormone oder RNA-Aptamere. In einer besonders bevorzugten Ausführungsform der erfindungsgemäßen Verwendung ist die Anwesenheit dieser Metallionen auf das Lumen der Liposomen beschränkt, um unspezifische Signale von außen anhaftendem und langsam freigesetztem Metallionen zu vermeiden. Zweckmäßig ist es auch, die Liposomen zur Herstellung von Nanokapseln zu verwenden. Mit Vorteil können die Liposomen zur Herstellung von Freisetzungssystemen in der Diagnostik verwendet werden. Zweckmäßig ist auch die Verwendung zum Transport und/oder zur Freisetzung von Wirkstoffen. Vorteilhafterweise können die Liposomen als Depotformulierung und/oder als zirkulierbares Depot verwendet werden. Vorteilhaft ist weiterhin die Verwendung der Liposomen als Vektor zur Transfektion von Zellen in vivo, in vitro und/oder

ex vivo. Die Liposomen sind beispielsweise bei intravenöser und/oder peritonealer Applikation verwendbar.

Die erfindungsgemäßen Sterolderivate und Liopsomen weisen mehrere Vorteile auf. Überraschenderweise konnte festgestellt werden, dass die Permeabilität der erfindungsgemäßen Liposomen vom pH-Wert und damit vom Ladungszustand des Sterolderivats abhängig ist. Wird beispielsweise Hist-Chol verwendet, so werden Liposomen umfassend ein Phosphatidylethanolamin bei einem pH-Wert von 5 bis 6 so permeabilisiert, dass eingeschlossene Wirkstoffe oder Marker innerhalb von Minuten bis Stunden ausdiffundieren. In anderen pH-Bereichen sind diese Membranen für sich genommen jedoch stabil und zeigen nur eine geringe Anfangspermeabilität. Liposomen unter Verwendung der erfindungsgemäßen Strukturen sind daher insbesondere geeignet zur Konstruktion von Freisetzungssystemen, bei denen eine Abgabe von Wirkstoffen in Abhängigkeit vom pH-Wert des Mediums erfolgen soll. Es wurde darüber hinaus überraschenderweise gefunden, dass in Liposomen, deren Membran die Sterolderivate umfasst, überdurchschnittlich hohe Mengen an Proteinen oder DNA eingeschlossen werden können. Die Effizienz dieses Einbaus ist dabei abhängig vom pH-Wert der verwendeten Lösung. Ein Prozess für die effiziente Verkapselung von Proteinen oder DNA in die Liposomen kann daher so geführt werden, dass zunächst ein pH-Wert eingestellt wird, der zu einer guten Bindung der Cargomoleküle an die Lipidschicht führt. Für DNA als Polyanion werden hier niedrige pH-Werte von etwa 4 bis 5 verwendet. Bei Proteinen richtet sich der nutzbare pH-Wert nach dem isoelektrischen Punkt des Proteins. Dieser sollte unterhalb des isoelektrischen Punktes der erfindungsgemäßen Substanz liegen. Eine Verkapselung ist dann besonders effektiv, wenn der pH-Wert des Mediums so gewählt wird, dass er zwischen dem isoelektrischen Punkt des Proteins und dem isoelektrischen Punkt des Sterolderivates liegt. Das Protein ist dann negativ und die Lipidschicht ist positiv geladen.

Weiterhin wurde überraschend gefunden, dass Liposomen, deren Membran beispielsweise Hist-Chol umfasst, zur Chelatisierung von Metallionen befähigt sind. Diese Eigenschaft führt zu einer Verstärkung der positiven Ladung des Liposoms. Dieser Effekt ist besonders stark bei neutralen pH-Werten zu beobachten, da dann die Eigenladung der Verbindung gering ist. Aufgrund ihrer chelatisierenden Eigenschaften lassen sich solche Liposomen in der biochemischen Diagnostik und zur pharmazeutischen Therapie verwenden.

Eine wesentliche Voraussetzung für die Verwendung von Liposomen für experimentelle oder therapeutische Zwecke ist deren Verträglichkeit mit Zellen und Geweben. Eine Reihe bekannter Verbindungen, die für das Einbringen von DNA oder Proteinen in Zellen genutzt werden (beispielsweise das kationische Lipid DOTAP) sind zytotoxisch. Es wurde überraschenderweise gefunden, dass einige der erfindungsgemäßen Verbindungen eine verringerte Zytotoxizität zeigen. Das betrifft insbesondere die Gruppe von Verbindungen, bei denen das Amphoter eine Aminosäure oder ein Peptid ist. Diese erfüllen daher eine der Voraussetzungen eines Transfektionssystems.

Eine weitere Voraussetzung für die Konstruktion von Vektoren zum Gen- oder Proteintransport in Zellen ist deren Kompatibilität mit Serum oder Blut. Wegen ihrer starken kationischen Ladung bilden die derzeit bekannten Vektoren mit Serum unkontrollierte große Aggregate, die im Organismus zur Bildung von Thromben führen. Ihre Verwendung ist damit in vivo praktisch ausgeschlossen und auf in vitro oder ex vivo Anwendungen beschränkt. Es wurde überraschenderweise gefunden, dass Liposomen, die unter Verwendung der erfindungsgemäßen Komponenten aufgebaut wurden, in Serum oder Blut keine Aggregate bilden. Das sind insbesondere solche Liposomen, deren isoelektrischen Punkt kleiner als 7,5 ist.

Eine weitere Voraussetzung für die Konstruktion von Vektoren zum Protein- oder Gentransfer ist deren Stabilität unter physiologischen Bedingungen. Liposomen werden bei einer Applikation in den Blutkreislauf vom Bestandteilen des Complementsystems angegriffen und schnell lysiert. Diese Reaktion erfolgt binnen Minuten. Es kommt zur Porenbildung in der Membran, durch die selbst große Moleküle wie Proteine ausdiffundieren können. Eine Stabilisierung von Liposomen gegenüber diesen Mechanismen ist bisher nur durch Einbau von Cholesterol in die Lipidschicht möglich. Solche Liposomen sind dann sehr stabil, können aber nicht mehr mit Zellen wechselwirken oder ihren Wirkstoff leicht abgeben. Es wurde überraschenderweise gefunden, dass Liposomen, die unter Verwendung der erfindungsgemäßen Komponenten aufgebaut werden, in Serum oder Blut über mehrere Stunden stabil sein können. Die Wirkstofffreisetzung ist auch unter diesen Bedingungen gering. Eine liposomaler Vektor für den Transport von Wirkstoffen muss mindestens drei Voraussetzungen erfüllen: er muss eine geringe Toxizität besitzen, den Wirkstoff sicher und stabil einschließen und kompatibel mit Serum oder Blut sein.

Alle drei dieser Voraussetzungen werden von Liposomen, die unter Verwendung ausgewählter erfindungsgemäßer Substanzen hergestellt sind, mit Vorteil erfüllt. Die Liposomen sind daher für eine Verwendung bei therapeutischen Zwecken gut geeignet. Weitere Eigenschaften, die diese Verwendung unterstützen, sind die gute Beladbarkeit mit Wirkstoffen und die gezielte Ablösung dieser Stoffe durch Veränderungen des pH-Werts oder durch Permeabilisierung der Membran. Liposomen, die unter Verwendung der erfindungsgemäßen Substanzen hergestellt werden, zeigen nur eine geringe unspezifische Bindung an Zelloberflächen. Diese geringe unspezifische Bindung ist eine wesentliche Voraussetzung für das Zustandekommen einer spezifischen Bindung an Zielzellen. Werden die beschriebenen Liposomen mit weiteren Liganden versehen, so ist eine Zielsteuerung der Vehikel gegeben. Der Wirkstoff kann dann spezifisch an solchen Zellen oder Geweben angereichert werden, die pathologische Zustände aufweisen.

Eine wesentliche Verwendung der erfindungsgemäßen Substanzen liegt daher bei der Konstruktion von Vektoren für den Wirkstofftransfer in lebenden Organismen. Die Vektoren sind besonders geeignet für den Transport von therapeutischen Makromolekülen wie etwa Proteinen oder DNA, die von sich aus nicht die Zellmembran überwinden können oder im Blutstrom schnell abgebaut werden.

Die Erfindung soll im folgenden anhand von Beispielen näher erläutert werden, ohne dass die Erfindung auf diese Beispiele zu beschränken ist.

**Beispiel 1**

**Synthese Cholesteryl-L-histidylsuccinat (Hist-chol)**

Stufe I, Aktivierung des Cholesterylhemisuccinates: 11,3 mmol (2,3 g) Dicyclohexylcarbodiimid werden in einem 100 ml Kolben mit 40 ml THF vorgelegt und auf -10°C gekühlt. 10,3 mmol (5 g) Cholesterinhemisuccinat und 11,3 mmol (1,3 g) N-Hydroxysuccinimid werden hinzugegeben. Man lässt die Mischung auf RT auftauen und rührt 5 h. Danach wird der gebildete Niederschlag abgetrennt (Harnstoff), das Filtrat eingeengt und der Rückstand aus Essigester umkristallisiert. Farblose Nadeln, mp: 145-146 °C, 93 %.

Stufe II, Cholesteryl-L-histidylsuccinate: 5,1 mmol (3 g) des aktivierten Esters (Stufe I) werden in 40 ml DMF vorgelegt. In 10 ml Wasser werden 7,6 mmol (0,6 g) Natriumhydrogencarbonat und 7,6 mmol (1,2 g) Histidin gelöst. Diese Lösung wird langsam zur DMF-Suspension getropft. Es wird 20 h gerührt und danach pH 4-5 mit 2N HCl eingestellt. Die Reaktionsmischung wird bis zur Trockene eingeengt und das Produkt mit heißem Ethanol extrahiert. Nach Abziehen des Ethanols fällt das Produkt rein an. Farbloser Feststoff, 78 %.

**Beispiel 2**

**Herstellung von amphoteren Liposomen**

5 mg Hist-Chol und 9.8 mg POPC werden in 4 mL Chloroform/Methanol (1:1 v/v) gelöst und im Rotationsverdampfer vollständig getrocknet. Der Lipidfilm wird mit 4.3 mL des entsprechenden Puffers (10 mM Kac, 10 mM HEPES, 150 mM NaCl, pH 7,5 in einer Lipidkonzentration von 5 mM durch 5 min Ultraschallbehandlung hydratisiert. Abschließend wird die Suspension eingefroren und nach dem Auftauen mehrfach extrudiert (Avestin LiposoFast, Polycarbonatfilter 200nm Porenweite). Der Verlauf des Zetapotentials in mV bei verschiedenen pH-Werten ist in der untenstehenden Tabelle 2 dargestellt.

Tabelle 2

| pH-Wert | 0mM NaCl | 100mM NaCl |
|---|---|---|
| 4,0 | +42 | -20 |
| 5,0 | +28 | +2 |
| 6,0 | -5 | -6 |
| 7,0 | -32 | -15 |
| 8,0 | -45 | -25 |

**Beispiel 3**

**Permeabilität**

Lipidfilme der Zusammensetzung DMPE/Hist-Chol 60: 40 mol% wurden anolog Beispiel 2 hergestellt und mit 100 mM 6-Carboxyfluorescein (CF), 50 mM NaCl, Hepes 10 mM, pH 8 hydratisiert, so dass eine Lipidkonzentration von 25 mM resultiert. Nichteingeschlossenes CF wurde durch Gelfiltration entfernt. Für die pH- und zeitabhängigen Permeabilitätsmessungen wurden die Liposomen auf 0,2 mM in Puffer des jeweiligen pHs verdünnt und nach 30 bzw. 60 min die Fluoreszenz gemessen. Dann wurde die Temperatur auf 37°C erhöht und nach den gleichen Zeitintervallen (30, 60 min) gemessen. Anschließend wurde die Temperatur auf 60°C erhöht und wieder nach 30 und 60 min gemessen. Die prozentuale Freisetzung von CF ist in der untenstehenden Tabelle 3 zusammengefaßt. Man erkennt deutlich eine Permeabilisierung bei pH 6,5, ein Wert, der dem isoelektrischen Punkt von Hist-Chol sehr nahe kommt. Oberhalb und unterhalb dieses pH-Wertes sind die Membranen überraschend stabil.

**Tabelle 3**

| | 0 min | 30 min | 60 min | 30 min (37°C) | 60 min (37°C) | 30 min (60°C) | 60 min (60°C) |
|---|---|---|---|---|---|---|---|
| PH 8.0 | 29% | 29% | 28% | 30% | 30% | 36% | 41% |
| pH 7.0 | 32% | 36% | 46% | 39% | 44% | 52% | 60% |
| pH 6.5 | 53% | 70% | 81% | 110% | 132% | 185% | 180% |
| pH 6.0 | 29% | 25% | 26% | 26% | 27% | 30% | 33% |
| pH 5.5 | 28% | 26% | 27% | 26% | 27% | 30% | 32% |
| pH 5.0 | 39% | 41% | 43% | 52% | 61% | 98% | 115% |

**Beispiel 4**

**Bindung von DNA an amphotere Liposomen**

Die in $KAc^{10}HEP^{10}NaCl^{100}$, pH 8, nach Beispiel 2 hergestellten Liposomen aus POPC/Hist-Chol 60:40 (mol%) wurden zur DNA-Bindung auf 0.2 mM mit dem zu untersuchenden Puffer verdünnt. DNA (HeringsDNA lmg/ml-Stammlösung in Wasser) wurde in entsprechenden Mengen vorgelegt. Anschließend wurde 1 ml 0.2 mM Liposomen zugegeben. Die Mischung wurde schnell gemischt und nach 15 min die Partikelgröße und das Zetapotential durch dynamische Lichtstreuung bestimmt. Bei richtig gewählter DNA-Menge bleibt die Partikelgröße fast unverändert.

Bei den pH-Werten 7 und 8, bei denen die POPC/HistChol 60:40 Liposomen negativ geladen sind, war keine Änderung in Größe bzw. Zetapotential zu erkennen, was eine Bindung von DNA ausschließt. Bei pH 4 und 5 sind die Liposomen stark positiv geladen und es kommt durch die DNA-Bindung zur Umladung der Partikel. Bei pH 6 sind die Liposomen nur schwach geladen, nach DNA-Zugabe stark negativ. Um diese Umladung zu erreichen muß erheblich mehr DNA addiert werden. Die Zetapotentiale und die dazugehörigen adsorbierten Mengen DNA sind in der untenstehenden Tabelle 4 zusammengefasst:

**Tabelle 4**

| pH-Wert | Zetapotential DNA (mV) | ohne/mit Adsorbierte Menge (μg/mg Lipid) | DNA |
|---|---|---|---|
| 4 | 41,3 | -37,7 | 15 |
| 5 | 19,6 | -44,7 | 15 |
| 6 | -4,2 | -36,7 | 40 |
| 7 | -36,8 | -35 | -- |
| 8 | -52,2 | -52,9 | -- |

Aus der Bindungscharakteristik kann ebenfalls abgeleitet werden, dass eine Ablösung nicht eingeschlossener, aussen anhaftender DNA durch Erhöhung des pH erreicht wird.

**Beispiel 5**

**Stabilität in Humanserum**

Liposomen der Zusammensetzung POPC/HistChol 60:40 wurden ananlog Beispiel 2 als 5 mM Suspension hergestellt. Der Serumtest wurde als Verdünnungsreihe durchgeführt.

Unterschiedliche Liposomenmengen (50 - 250 μl) wurden mit dem gleichen Volumen Humanserum (250μl) für 5 min bei 37°C inkubiert (das Gesamtvolumen wurde durch Addition von 150 mM NaCl immer auf konstant 500μl gebracht). Zur Messung der Partikelgröße mit dynamischer Lichtstreuung wurde die Mischung dann 1:9 mit Puffer (KAc10 Hep10 NaCl100; pH 8) verdünnt. Die Daten sind in der untenstehenden Tabelle zusammengestellt. Die gemessene Partikelgröße nähert sich dem Serumwert an, je konzentrierter das Serum ist. Die Bildung von großen (> 1μm) Aggregaten wurde nicht beobachtet (Tabelle 5).

**Tabelle 5**

| Probe 250μl Serum + x μl Liposomen | Partikelgröße (nm) | Polydispersität |
|---|---|---|
| 250 | 94 | 0,574 |

(fortgesetzt)

| Probe 250μl Serum + x μl Liposomen | Partikelgröße (nm) | Polydispersität |
|---|---|---|
| 125 | 89 | 0,584 |
| 50 | 81 | 0,589 |
| Nur Serum | 70 | 0,613 |
| Nur Liposomen | 119 | 0,155 |

**Beispiel 6**

**Pharmakokinetik im Blut**

Je 500 μL Liposomen aus POPC/Chol und POPC/Hist-Chol wurden männlichen Wistar-Ratten per Injektion in die Schwanzvene verabreicht.

50 mM Liposomen-Suspensionen wurden hergestellt durch Hydratisieren eines Lipidfilms der entsprechenden Formulierung (Addition von 0,03 mol% [14]C-DPPC) mit 2 mL einer Lösung von 1 mg [3]H-Inulin in HEPES 10 mM, NaCL 150 mM, pH 8). Nach 3 Einfrier/Auftau-zyklen wurden die Suspensionen durch eine 400 nm-Membran mehrfach extrudiert (LiposoFast, Avestin). Abtrennung von nichteingeschlossenem [3]H-Inulin erfolgte durch Gelfitration über eine G-75 Sephadex-Säule und anschließende Konzentrierung über CENTRIPREP (Millipore) Zentrifugationseinheiten.4 Versuchstieren je Formulierung wurden 0,5 mL Liposomensuspension verabreicht und Blutproben nach 5 min,15 min, 60 min, 3 h, 12 h, 24 h genommen. Ca. 50 -100 mg der Blutproben wurden in 1 mL SOLVABLE-Gewebelöser (PACKARD) bei 50°C für 1-3 h aufgelöst und anschließend mit 0,1-0,5 mL 30% Wasserstoffperoxyd-Lösung entfärbt. Danach wurden 10 mL Szintillator hinzugefügt und die Aktivität von [3]H und [14]C gemessen. Eine unmittelbare toxische Wirkung der Verbindungen war nicht nachzuweisen.

| Eliminationshalbwertszeiten aus dem Blut: | |
|---|---|
| POPC/Chol | größer 120 min |
| POPC/Hist-Chol | größer 90 min |

**Patentansprüche**

1. Sterolderivat nach der allgemeinen Formel (1):

$$\text{Amphoter - Y - Spacer - X - Sterol} \qquad (1),$$

wobei
das Amphoter einen oder mehrere kationische Ladungsteile und einen oder mehrere anionische Ladungsteile umfasst, weiterhin

- der kationische Ladungsteil aus der Gruppe enthaltend Piperazine, Imidazole, Morpholine, Purine und/oder Pyrimidinen ausgewählt wird, dessen pKa-Wert zwischen 4 und 8 liegt, und der anionische Ladungsteil eine Carboxylgruppe enthält, dessen pKa-Wert zwischen 3 und 7 liegt, und
- der Spacer ausgewählt aus der Gruppe enthaltend Niederalkylreste mit bis zu 8 C-Atomen mit linearer, verzweigter oder ringförmiger Struktur und 0, 1 oder 2 ethylenisch ungesättigten Bindungen und
- die verbindenden Gruppen X und/oder Y ausgewählt sind aus der Gruppe bestehend aus -(C=O)-O-; -(C=O)-NH-; -NH-(C=O)-O-; -(C=O)-S-; -O-; -NH-; -S-; - CH=N-; -0-(O=C)-; -S-(0=C)-; -NH-(O=C)-; -O-(C=O)-NH- und/oder -N=CH- und
- das Sterol ausgewählt ist aus der Gruppe bestehend aus Cholesterol, Sitosterol, Campesterol, Desmosterol, Fucosterol, 22-Ketosterol, 20-Hydroxysterol, Stigmasterol, 22-Hydroxycholesterol, 25-Hydroxycholesterol, Lanosterol, 7-Dehydrocholesterol, Dihydrocholesterol, 19-Hydroxycholesterol, 5α-Cholest-7-en-3β-ol, 7-Hydroxycholesterol, Epicholesterol, Ergosterol und/oder Dehydroergosterol und
- das Gesamtmolekül einen isoelektrischen Punkt zwischen 4,5 und 8,5 aufweist.

2. Sterolderivat nach einem der vorgehenden Ansprüche,

**dadurch gekenntzeichnet, dass**
das Gesamtmolekül einen isoelektrischen Punkt zwischen 5 und 7 aufweist.

3. Liposomen umfassend Sterolderivate nach Anspruch 1 oder 2.

4. Liposomen nach dem vorhergehenden Anspruch, **dadurch gekennzeichnet, dass** die Liposomen maximal 50 mol% des Sterolderivats umfassen, bevorzugt 2 bis 50 mol%, besonders bevorzugt 10 bis 40 mol%.

5. Liposomen nach Anspruch 3 oder 4, **dadurch gekennzeichnet, dass** die Liposomen Phophatidylcholin, Phosphatidylethanolamin, Diacylglycerol, Tetraetherlipid und/oder PEG-Lipid umfassen.

6. Liposomen nach einem der Ansprüche 3 bis 5, **dadurch gekennzeichnet**, das die Liposomen eine mittlere Größe zwischen 50 und 1000 nm, bevorzugt zwischen 50 und 300 nm, besonders bevorzugt zwischen 60 und 130 nm aufweisen.

7. Liposomen nach einem der Ansprüche 3 bis 6, **dadurch gekennzeichnet, dass** die Liposomen einen Wirkstoff umfassen.

8. Liposomen nach einem der Ansprüche 3 bis 7, **dadurch gekennzeichnet, dass** der Wirkstoff ein Protein, ein Peptid, eine DNA, eine RNA, ein antisense-Nukleotid und/oder ein Decoy-Nukleotid ist.

9. Liposomen nach Anspruch 7 oder 8, **dadurch gekennzeichnet, dass** mindestens 80% des Wirkstoffes im Innern des Liposoms eingeschlossen sind.

10. Verfahren zur Wirkstoffbeladung von Liposomen nach einem der Ansprüche 3 bis 9, **dadurch gekennzeichnet, dass** ein Bindungs-pH-Wert zur Verkapselung von Wirkstoffen eingesetzt wird und ein zweiter pH-Wert zur Abtrennung der nicht gebundenen Wirkstoffe verwendet wird.

11. Verfahren zur Wirkstoffbeladung von Liposomen nach einem der Ansprüche 3 bis 9, **dadurch gekennzeichnet, dass** die Liposomen bei einem bestimmten pH-Wert permeabilisiert und folgend verschlossen werden.

12. Verwendung der Liposomen nach einem der Ansprüche 3 bis 9 zur Herstellung von Nanokapseln.

13. Verwendung der Liposomen nach einem der Ansprüche 3 bis 9 zur Herstellung von Freisetzungssystemen in der Diagnostik.

14. Verwendung der Liposomen nach einem der Ansprüche 3 bis 9 zur Herstellung von pharmazeutischen Präparaten zum Transport und zur Freisetzung von Wirkstoffen.

15. Verwendung der Liposomen nach einem der Ansprüche 3 bis 9 zur Herstellung von pharmazeutischen Präparaten, die zur Depotformulierung und/oder als zirkulierbares Depot dienen.

16. Verwendung der Liposomen nach einem der Ansprüche 3 bis 9 zur Herstellung von pharmazeutischen Präparaten, die als Vektor zur Transfektion von Zellen in vivo, in vitro und/oder ex vivo dienen.

17. Verwendung der Liposomen nach einem der Ansprüche 3 bis 9 zur Herstellung von pharmazeutischen Präparaten zur intravenösen und/oder peritonealen Applikation.

## Claims

1. A sterol derivative according to general formula (1):

Amphoteric substance - Y - spacer - X - sterol (1),

wherein

the amphoteric substance comprises one or more portions of cationic charge and one or more portions of anionic charge,
and

- the portion of cationic charge being selected from the group consisting of piperazines, imidazoles, morpholines, purines and/or pyrimidines, with a pKa value between 4 and 8 and the portion of anionic charge including a carboxyl group, with a pKa value between 3 and 7 and
- the spacer being selected from the group consisting of lower alkyl residues with up to 8 C atoms and with a linear, branched or cyclic structure and 0, 1 or 2 ethylenically unsaturated bonds, and
- the linking groups X and/or Y being selected from the group consisting of -(C=O)-O-, -(C=O)-NH-, -NH-(C=O)-O-, -(C=O)-S-, -O-, -NH-, -S-, -CH=N-, -O-(O=C)-, -S-(O=C)-, -NH-(O=C)-, -O-(O=C)-NH- and/or -N=CH-, and
- the sterol being selected from the group consisting of cholesterol, sitosterol, campesterol, desmosterol, fucosterol, 22-ketosterol, 20-hydroxysterol, stigmasterol, 22-hydroxycholesterol, 25-hydroxycholesterol, lanosterol, 7-dehydrocholesterol, dihydrocholesterol, 19-hydroxycholesterol, 5α-cholest-7-en-3β-ol, 7-hydroxycholesterol, epicholesterol, ergosterol, and/or dehydroergosterol, and
- the overall molecule having an isoelectric point of between 4.5 and 8.5.

**2.** The sterol derivative according to any of the preceding claims, **characterized in that** the overall molecule has an isoelectric point of between 5 and 7.

**3.** Liposomes comprising the sterol derivative according to any of claim 1 or 2.

**4.** The liposomes according to the preceding claim, **characterized in that** the liposomes comprise 50 mole-% of sterol derivative at maximum, and preferably from 2 to 50 mole-%, more preferably from 10 to 40 mole-%.

**5.** The liposomes according to claim 3 or 4, **characterized in that** the liposomes comprise phosphatidyl choline, phosphatidyl ethanolamine, diacylglycerol, tetraether lipid and/or PEG lipid.

**6.** The liposomes according to any of claims 3 to 5, **characterized in that** the liposomes have an average size of between 50 and 1000 nm, preferably between 50 and 300 nm, and more preferably between 60 and 130 nm.

**7.** The liposomes according to any of claims 3 to 6, **characterized in that** the liposomes comprise an active substance.

**8.** The liposomes according to any of claims 3 to 7, **characterized in that** the active substance is a protein, a peptide, a DNA, an RNA, an antisense nucleotide and/or a decoy nucleotide.

**9.** The liposomes according to claim 7 or 8, **characterized in that** at least 80% of the active substance is entrapped inside the liposome.

**10.** A method of loading liposomes according to any of claims 3 to 9 with active substances, **characterized in that** a binding pH value for encapsulation of active substances and a second pH value for removal of unbound active substances is used.

**11.** A method of loading liposomes according to any of claims 3 to 9 with active substances, **characterized in that** the liposomes are made permeable at a specific pH value and subsequently sealed.

**12.** Use of the liposomes according to any of claims 3 to 9 in the production of nanocapsules.

**13.** Use of the liposomes according to any of claims 3 to 9 in the production of release systems in diagnostics.

**14.** Use of the liposomes according to any of claims 3 to 9 in the production of pharmaceutical preparations for the transport and release of active substances.

**15.** Use of the liposomes according to any of claims 3 to 9 in the production of pharmaceutical preparations used as depot formulation and/or as circulative depot.

**16.** Use of the liposomes according to any of claims 3 to 9 in the production of pharmaceutical preparations used as a

vector to transfect cells *in vivo,* in vitro and/or *ex vivo.*

**17.** Use of the liposomes according to any of claims 3 to 9 in the production of pharmaceutical preparations for intravenous and/or peritoneal application.

**Revendications**

**1.** Dérivé de stérol selon la formule générale (1) :

amphotère – Y – espaceur – X – stérol (1) ,

dans laquelle
l'amphotère comprend une ou plusieurs parties de charge cationiques et une ou plusieurs parties de charge anioniques,
et en outre

- la partie de charge cationique est sélectionnée parmi le groupe comprenant les pipérazines, les imidazoles, les morpholines, les purines et/ou les pyrimidines, dont le pKa est compris entre 4 et 8, et la partie de charge anionique contient un groupe carboxyle, dont le pKa est entre 3 et 7, et
- l'espaceur est sélectionné parmi le groupe comprenant les radicaux alkyle inférieurs comprenant jusqu'à 8 atomes de C, ayant une structure linéaire, ramifiée ou cyclique et comportant 0, 1 ou 2 liaisons éthylèniquement insaturées et
- les groupes liants X et/ou Y sont sélectionnés dans le groupe constitué de -(C=O)-O-, -(C=O)-NH-, -NH-(C=O)-O-, -(C=O)-S-, -O-, -NH-, -S-, -CH=N-, -O-(O=C)-, -S-(O=C)-, -NH-(O=C)-, -O-(C=O)-NH- et/ou -N=CH- et
- le stérol est choisi dans le groupe comprenant le cholestérol, le sitostérol, le campestérol, le désmostérol, le fucostérol, le 22-cétostérol, le 20-hydroxystérol, le stigmastérol, le 22-hydroxycholestérol, le 25-hydroxycholestérol, le lanostérol, le 7-déhydrocholestérol, le dihydrocholestérol, le 19-hydroxycholestérol, le 5α-cholest-7-en-3β-ol, le 7-hydroxycholestérol, l'épicholestérol, l'ergostérol et/ou le déhydroergostérol et
- la molécule entière a un point isoélectrique entre 4,5 et 8,5.

**2.** Dérivé de stérol selon la revendication précédente,
**caractérisé en ce que**
la molécule entière a un point isoélectrique entre 5 et 7.

**3.** Liposomes comprenant des dérivés de stérol selon la revendication 1 ou 2.

**4.** Liposomes selon la revendication précédente, **caractérisés en ce que** les liposomes comprennent au maximum 50 % en moles du dérivé de stérol, de préférence de 2 à 50 % en moles et de manière encore plus préférée de 10 à 40 % en moles.

**5.** Liposomes selon la revendication 3 ou 4, **caractérisés en ce que** les liposomes comprennent la phosphatidylcholine, le phosphatidyléthanolamine, le diacylglycérol, le tétraétherlipide et/ou les lipides PEG.

**6.** Liposomes selon l'une des revendications 3 à 5, **caractérisés en ce que** les liposomes ont une grandeur moyenne entre 50 et 1000 nm, de préférence entre 50 et 300 nm, et de manière encore plus préférée entre 60 et 130 nm.

**7.** Liposomes selon l'une des revendications 3 à 6, **caractérisés en ce que** les liposomes contiennent un principe actif.

**8.** Liposomes selon l'une des revendications 3 à 7, **caractérisés en ce que** le principe actif est une protéine, un peptide, un ADN, un ARN, un nucléotide antisens et/ou un nucléotide decoy.

**9.** Liposomes selon la revendication 7 ou 8, **caractérisés en ce qu'**au moins 80 % du principe actif sont incorporés à l'intérieur du liposome.

**10.** Procédé de charge de principe actif dans les liposomes selon l'une des revendications 3 à 9, **caractérisé en ce qu'**on utilise un certain pH de liaison pour encapsuler les principes actifs et qu'on utilise un deuxième pH pour séparer les principes actifs non liés.

**11.** Procédé de charge de principe actif dans les liposomes selon l'une des revendications 3 à 9, **caractérisé en ce qu'**on rend perméables les liposomes à un certain pH et qu'on les obture ensuite.

**12.** Utilisation des liposomes selon l'une des revendications 3 à 9 pour préparer des nanocapsules.

**13.** Utilisation des liposomes selon l'une des revendications 3 à 9 pour préparer des systèmes de libération dans le diagnostic.

**14.** Utilisation des liposomes selon l'une des revendications 3 à 9 pour préparer des préparations pharmaceutiques destinées au transport et à la libération de principes actifs.

**15.** Utilisation des liposomes selon l'une des revendications 3 à 9 pour préparer des préparations pharmaceutiques destinées à une formulation de retard et/ou une forme retard pouvant circuler.

**16.** Utilisation des liposomes selon l'une des revendications 3 à 9 pour préparer des préparations pharmaceutiques, qui servent de vecteur de transfection de cellules in vivo, in vitro et/ou ex vivo.

**17.** Utilisation des liposomes selon l'une des revendications 3 à 9 pour préparer des préparations pharmaceutiques destinées à une administration par voie intraveineuse et/ou péritonéale.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4891208 A
- WO 0059474 A
- WO 0028972 A
- WO 0164330 A


### In der Beschreibung aufgeführte Nicht-Patentliteratur


- **TACHIBANA et al.** *BBRC,* 1998, vol. 251, 538-544
- *Handbook of Chemistry and Physics,* vol. 73, 8-37